# EUROPEAN PATENT APPLICATION

(11) **EP 2 740 436 A1**
(43) Date of publication of application: **11.06.2014**
(21) Application number: 13005645.0
(22) Date of filing: 04.12.2013
(51) Int. Cl.: A61B 19/04, B29C 41/38

(54) **Surgical glove**

(30) Priority: 05.12.2012 MY PI2012005261
(71) Applicant: Low, Chin Guan, 16100 Kota Bharu, Kelantan (MY)
(72) Inventor: Guan Chin Low, 16100 Kota Bharu (MY); Guan, Chin Low, 16100 Kota Bharu (MY)
(74) Representative: Angerhausen, Christoph

(57) **Abstract**

A surgical glove is made using a mould having a set of digits, consisting of thumb and index, middle, ring and little fingers that are disposed In positions consistent with that of a human hand when holding a surgical implement. In one embodiment, the digits include at least one major bend. In another embodiment, each of the digits includes two bends. By adopting a shape for the surgical glove that mimics the position of the surgeon's hand while holding a surgical implement, finger stress and hand fatigue during a prolonged surgical procedure can be significantly reduced.

## Description

### FIELD OF INVENTION

The invention relates generally to a surgical glove and to a hand mould for making such a glove. More specifically, the invention concerns a surgical glove which may be worn by a surgical doctor for an extended period of time while performing surgery.

### BACKGROUND OF THE INVENTION

In the course of a surgical procedure, a typical surgical glove is worn while the doctor is performing a surgery in a surgery room. A typical surgical glove can be worn from as little as 10 minutes to more than 15 hours to complete a procedure. In the course of a long surgery, many pairs of gloves are changed for cleanliness, leaving the surgical room for rest or food, good sterile practice, etc. Nevertheless the amount of time when the gloves are worn on the hands is long enough to cause hand fatigue. Such length of time puts a physical stress on the hands of the surgeon as the gloves are designed to be worn tight without slack or loose folds in order to maximize dexterity and sensitivity. Being elastic in nature, all surgical gloves exert a certain amount of pressure on the hands, the amount varying according to the type of materials, the elastic compression, the dimensions of the gloves or hands, and the shape of the gloves compared to the shape of the hands. The last factor is crucial as any shape that is not conforming to the shape of the hand will exert a certain amount of stress on the part of the hand that is out of conformance to the gloves. Hand fatigue is a major issue for surgeons due to the protracted length of some of these procedures. As such, methods of reducing hand fatigue, which is caused primarily by the fact that surgical gloves are worn tight to increase dexterity and sensitivity, are important factors in the design of surgical glove.

GB-A-2148094 discloses a plastic glove made by dipping a hand-shaped form which comprises an index finger portion, a middle finger portion, a third finger portion and a little finger portion, each defining a curved line extending from the knuckle portion to the fingertip portion with an angle of curvature of from 35° to 90°. Preferably there is an included angle of less than 90° between a first tangent touching the curved line at the fingertip portion and a second tangent touching on the back side surface of the hand portion.

WO-A-98/14079 discloses an anatomically-accurate surgical glove manufactured from an elastomeric material, having an enlarged thumb ball portion and independenfily curved fingers.

Most surgical gloves today come in two shapes which are either with straight fingers or slightly curved fingers, the latter exemplified by WO-A-98/14079. This slightly curved shape is illustrated in Fig. 1, However these shapes do not address or adequately address the problem of finger stress and the related hand fatigue. No-one, including surgeons, keeps their hands open with the fingers straight or slightly curved in a smooth parabolic line.

There is thus an unfulfilled need for a surgical glove that is able to address the hand fatigue problems faced by those who wear the surgical glove for a long period of time while working with surgical implements. The present invention was developed in consideration of this need.

### SUMMARY OF THE INVENTION

In a first aspect, the invention provides a surgical glove hand mould comprising a set of digits, consisting of thumb and index, middle, ring and little fingers, wherein the digits are disposed in positions consistent with that of a human hand when holding a surgical implement.

In one embodiment, each of the digits includes at least one major bend.

In one specific embodiment, the digits of the mould are to copy the one major bend (Bend 1) of the proximal-middle joint for the index, middle, ring and little fingers. For the thumb, the one major bend (Bend 1) occurs at the metacarpal-proximal joint.

The location of Bend 1 of the fingers and thumb is defined as the point of the first bend measured from the base of the finger crouch as a percentage of the total length of the straightened finger from finger crouch to fingertip. An angle of bend (a) of the first bend of the fingers is defined as the acute angle formed by the intersection of a first line formed along the proximal bone and a second line formed along the middle bone. An angle of bend (a) of the first bend for the thumb is defined as the acute angle formed by the intersection of a first line formed along the metacarpal bone and a second line formed along the proximal bone.

In another embodiment, each of the digits includes two bends.

In one embodiment, the locations of Bend 1 are as per Table 1 herein.

| | **Location of Bend 1** | **Angle of Bend (a)** |
|---|---|---|
| Thumb | 0-40% | 0-30 degrees |
| Index | 20-40% | 35-65 degrees |
| Middle | 25-45% | 35-65 degrees |
| Ring | 25-45% | 35-65 degrees |
| Little | 25-45% | 35-65 degrees |

In an embodiment, the digits of the mould are to copy the two bends of the proximal-middle joint and middle-distal bone joint for the index, middle, ring and little fingers. For the thumb, the first bend (Bend 1) occurs at the metacarpal-proximal joint and the second bend (Bend 2) occurs at the proximal-distal joint.

The location of Bend 2 of the fingers and thumb is defined as the point of the second bend measured from the base of the finger crouch as a percentage of the total length of the straightened finger from finger crouch to fingertip. An angle of bend (b) of the second bend of the fingers is defined as the acute angle formed by the intersection of a first line formed along the middle bone and a second line formed along the distal bone. An angle of bend (b) of the second bend is defined as the acute angle formed by the intersection of a first line formed along the proximal bone and a second line formed along the distal bone.

In one embodiment, the locations of Bend 1 and Bend 2 are as per Table 2 herein.

| | **Location of Bend 1** | **Location of Bend 2** | **Angle of Bend (b)** | **Combined Aggregate Angle of Bend (c) from the Metacarpal Bone Line** |
|---|---|---|---|---|
| Thumb | 0-45% | 0-80% | 0-30 degrees | 0-45 degrees |
| Index | 25-45% | 60-80% | 30-60 degrees | 31-90 degrees |
| Middle | 25-45% | 60-80% | 30-60 degrees | 31-90 degrees |
| Ring | 25-45% | 60-80% | 30-60 degrees | 31-90 degrees |
| Little | 25-45% | 60-80% | 30-60 degrees | 31-90 degrees |

In a further embodiment, the thumb and index finger are relatively disposed to define a gap between them of approximately 1 cm,

In a second aspect, the invention provides a surgical glove formed using the mould according to the first aspect.

In an embodiment, each of the digits of the surgical glove includes at least one major bend. A first bend of the fingers is located at a proximal-middle joint. A first bend of the thumb is located at a metacarpal-proximal joint.

In another embodiment, each of the digits of the surgical glove includes two bends. A first bend of the fingers is located at a proximal-middle joint and the second bend of the fingers is located at a middle-distal joint. A first bend of the thumb is located at a metacarpal-proximal joint and the second bend of the thumb is located at a proximal-distal joint.

The glove mould or former may be made of ceramic, porcelain, aluminium, cast metal, polymers, or polymer-based material. The glove is formed by dipping the mould into a bath of liquid latex, which is then dried and vulcanized by heating to a temperature sufficient to crosslink the latex molecules. The gloves are stripped from the mould by hand or machine, individually in a two-dimensional movement.

The surgical glove may be made of any of natural latex, polyurethane, synthetic latex, polyisoprene latex, chloroprene latex, acrylonitrile butadiene based latex, and blends or combinations of these latex, PVC, SEBS, SIS and variants or combinations of these materials with any other latex(s) mentioned above.

The present invention seeks to provide a solution to the hand fatigue problems by introducing a particular and one or more distinctive bends in the fingers of the glove. Generally, the strain on a surgeon's hands is caused by the fact that the surgeon's hands are not posed in a way which resembles the pose of the hands when in rested position. In most cases, the surgeon will be holding a tool of some sort and so will have his hands almost closed. To overcome the tendency of the glove to return to its natural shape, one which approximates the shape of the hand when in rested position that the glove was produced with, the surgeon must exert continual force on the glove to grasp the tool. The present invention overcomes this limitation by creating a mould that, when used to make gloves, creates stress and slack in such a way that the natural shape of the glove when worn will approximate the pose of the surgeon's hands while manipulating a surgical implement.

The present invention thus reduces tension in the glove by using dimensional proportions that match closely the hand position most commonly occupied by a surgeon while retaining the tightness which provides improved sensitivity and dexterity. Unlike the above prior art that provides a gentle curve to the digits of the glove, the present invention is based on the idea of specific articulation required during the process of surgery including positioning of one or more bends in the former used to produce the gloves.

### BRIEF DESCRIPTION OF THE DRAWINGS

The present invention is illustrated, though not limited, by the following description of embodiments that are given by way of example only in conjunction with the accompanying drawings, in which:
Figure 1 is shows a prior art glove mould having a slight curvature;
Figure 2 shows the bones of a human hand;
Figure 3 shows the natural position of a surgeon's hand that is already in a casual folder disposition.
Figure 4 shows the natural position of a surgeon's hand in a grip position when holding a surgical implement;
Figure 5 shows the measurement of the location of Bend 1 and Bend 2 in the form of percentage (%) on the finger joints.
Figure 6 shows the first embodiment in which the mould has one major bend to copy;and
Figure 7 shows the first embodiment in which the mould has two major bends to copy.

### DETAILED DESCRIPTION OF THE EMBODIMENTS

If one is to wear a glove with fingers straight up from the palm, and the hand is to be folded into a fist, the biggest change in shape will be the fingers, followed a slight deformation of the back of hand. In this instance, if the hand is to be folded into a fist and held on in that position for a certain period of time, the fingers will start to tire and feel stressed to continue being folded into a fist due to the stress and pressure of the glove to unfold back to a straight finger pose.

In order to reduce this stress, the material of the glove can be made softer to reduce the elastic stress being exerted when deformed/stretched, However, there is a limit to this as a material that is too elastic will not function as a skin tight glove, easily stretching and will be unable to return to shape quickly.

Recognizing that given any material, a substantial amount of finger stress is actually coming from the amount of deformation, hence the elastic stress to return to shape, the present invention proposes to make a glove that conforms to the disposition of the hand of the surgeon most of the time when he is actually wearing the glove and at work on a surgical procedure.

The surgeon's hands can be in several poses: hands down by his side (very seldom), hands lifted to waist length with hands forward (very often) and using this same pose with the hands gripping something, be it a scalpel, tool, etc. (most common).

The invention is thus to address this position of the hands that is very often the position of the surgeon's hands when in surgery.

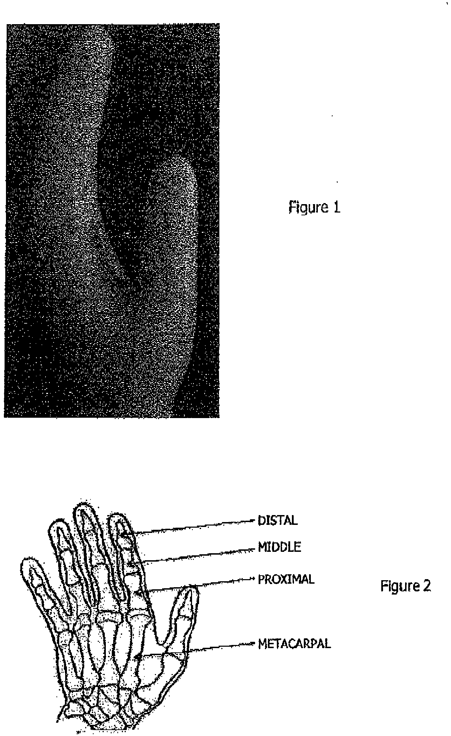

Most surgical gloves commonly available to the public come in the shape of slightly curved fingers glove as illustrated by Figure 1. The glove as illustrated in Figure 1 does not address the finger stress as experienced by the surgeon and hence does not make effective glove.

Figure 2 illustrates the four major bones of a human hand namely the metacarpal, proximal, middle and distal bones of the fingers.

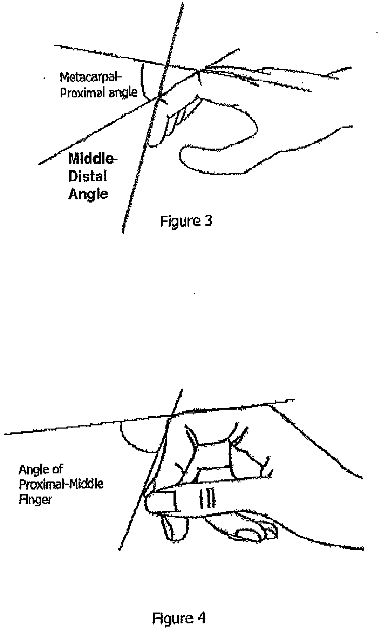

The invention is to make a glove that is shaped like a hand that is already in a casual folded disposition, as shown in Figure 3. As described above, this is also the pose of the hand which a surgeon uses the most when working. Further observation is that the surgeon's hand is not totally dosed where the index finger is touching the thumb, but more often in a position that is holding something of about 1 cm in size between his index finger and thumb.

An embodiment of the invention as illustrated by Figure 4 thus provides a shape of glove that closely matches this arrangement of fingers-palm-thumb relative positions in which the hand is in a grip position.

A typical range of angles of the fingers in the various positions are as follows, with Figures 3 and 4 illustrating how these angles can be measured.

| | Metacarpal-Proximal | Proximal-Middle | Middle-Distal |
|---|---|---|---|
| Hand in Casual Position | 25-45° | 25-40° | 10-18° |
| Hand in Grip Position | 45-65° | 40-50° | 28-40° |

The angles vary depending on the ratio of the finger bones and also according to gender.

| | Distal | Middle | Proximal | Metacarpal |
|---|---|---|---|---|
| Index | 1 | 1.1-1.4 | 1.8-2.8 | 3.2-4.3 |
| Middle | 1 | 1.3-1.8 | 2.2-2.7 | 3.0-3.9 |
| Ring | 1 | 1.3-1.7 | 2.0-2.8 | 3.0-3.6 |

The invention takes into account the ratio of lengths of the fingers and the angles to produce a shape that is as close to the shape of the hand at those positions.

If we observe our own hands, most of the time our fingers are bent at two to three positions. Starting from the metacarpal, the first bend is at the metacarpal-proximal joint. The second bend is at the proximal-middle joint, and the third bend is at the middle-distal digit joint. The two major bends occur at the proximal-middle joint and the middle-distal joint.

The thumb does not have a middle bone. For the thumb, the two major bends occur at the metacarpal-proximal joint and the proximal-distal joint.

The locations of the bends are related and relative to the finger lengths. Typically surgical gloves are made to sizes of 5, 5,5, 6, 6.5, 7, 7.5, 8, 8.5, 9, 9.5 and 10. There are many finger lengths that can be designed and made without the need for compliance to any standards, international, regional or national. The only criterion is to attempt to match the general demographic anatomical size, with certain regions of the world preferring shorter fingers in a glove and others preferring generally longer fingers in a glove. This also depends on the size of the glove as described above.

The only parameter to which any international, regional or national standards apply is the width of the palm that is to match the sizes. The relevant standards are ASTM 3577, EN 455 and ISO 11232. These sizes again do not have any fixed standard finger lengths.

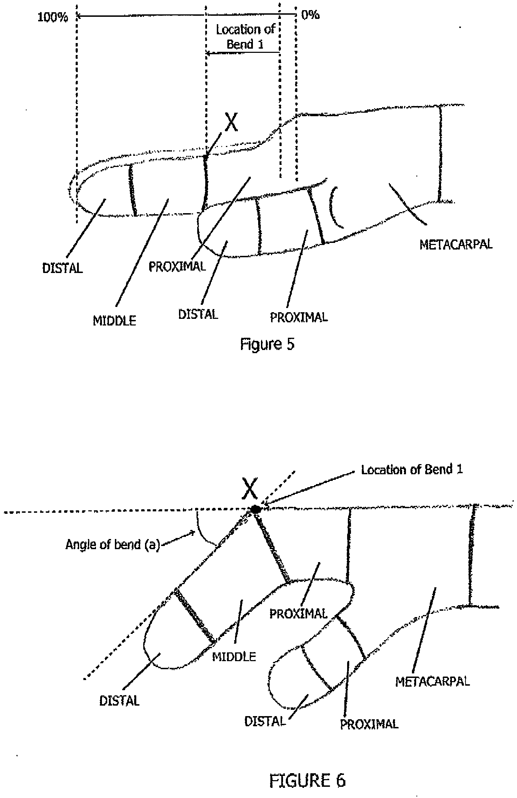

Figures 5 to 7 show how the location of Bend 1 represented by point X and the location of Bend 2 represented by point Y on the finger digits are measured in the form of percentage (%) of the total length of the straightened finger from finger crouch to fingertip respectively. The locations of Bends 1 and 2 for the five fingers are measured from the base of the finger crouches (knuckle of the respective fingers) to the respective point of bends when the fingers are in straightened positions as seen from Figure 5.100% thus corresponds to the fingertip location and 0% to the location of the knuckle.

The first embodiment of the invention resides in a mould that has one major bend (Bend 1) in the digits as illustrated in Figure 6 at the point X. This is to copy the one major bend (Bend 1) of the proximal-middle joint for the index, middle, ring and little fingers. For the thumb, the one major bend (Bend 1) occurs at the metacarpal-proximal joint.

In order to be suitable for any design of finger lengths, regardless of size, the percentages / ratios and angles rather than absolute dimensions are specified in Table 1. Figure 6 illustrates how the parameters in Table 1 of the first bend in the form of location of Bend 1 (X) and angle of bend (a) are measured. The range of locations of Bend 1 (X) in percentage (%) for the different parts of the fingers as tabulated in Table 1 are based on general demographic anatomical sizes.

The angle of bend (a) of the first bend for the index, middle, ring and little fingers is defined as the acute angle formed by the intersection of a first line formed along the proximal bone and a second line formed along the middle bone.

The angle of bend (a) of the first bend for the thumb is defined as the acute angle formed by the intersection of a first line formed along the metacarpal bone and a second line formed along the proximal bone.

**Table 1**

| | **Location of Bend 1** | **Angle of Bend (a)** |
|---|---|---|
| Thumb | 0-40% | 0-30 degrees |
| Index | 20-40% | 35-65 degrees |
| Middle | 25-45% | 35-65 degrees |
| Ring | 25-45% | 35-65 degrees |
| Little | 25-45% | 35-65 degrees |

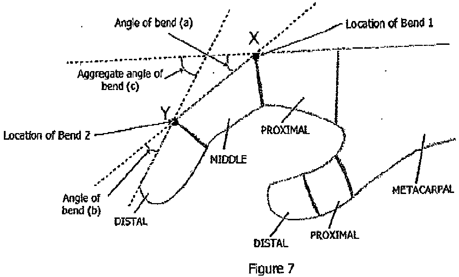

The second embodiment of the Invention resides in a mould that has two major bends In the digits as illustrated In Figure 7. This is to copy the two bends of the proxlmal-middle joint and middle-distal bone joint for the index, middle, ring and little fingers. For the thumb, the first bend (Bend 1) occurs at the metacarpal-proximal joint and the second bend (Bend 2) occurs at the proximat-distal joint.

In this second embodiment, the aggregate of the two bends from the metacarpal line will be the defining angle as measured as angle of bend (c). See Table 2. Figure 7 illustrates how the parameters as tabulated in Table 2 of the two bends in the form of location of Bend 1 represented by point X and angle of bend (a) and location of Bend 2 represented by point Y and angle of bend (b) are measured. The measurement of the angle of the aggregate angle of bend (c) is also shown. The range of locations of Bend 1 (X) and Bend 2 in percentage (%) for the different parts of the fingers as tabulated in Table 2 are based on general demographic anatomical sizes.

The angle of bend (b) of the second bend for the index, middle, ring and little fingers is defined as the acute angle formed by the intersection of a first line formed along the middle bone and a second line formed along the distal bone. The angle of bend (c) is the combined aggregate angles of bend (a) and bend (b).

The angle of bend (b) of the second bend for the thumb is defined as the acute angle formed by the intersection of a first line formed along the proximal bone and a second line formed along the distal bone. The angle of bend (c) is the combined aggregate angles of bend (a) and bend (b).

**Table 2**

| | **Location of Bend 1** | **Location of Bend 2** | **Angle of Bend (b)** | **Combined Aggregate Angle of Bend (c) from the Metacarpal Bone Line** |
|---|---|---|---|---|
| Thumb | 0-45% | 0-80% | 0-30 degrees | 0-45 degrees |
| Index | 25-45% | 60-80% | 30-60 degrees | 31-90 degrees |
| Middle | 25-45% | 60-80% | 30-60 degrees | 31-90 degrees |
| Ring | 25-45% | 60-80% | 30-60 degrees | 31-90 degrees |
| Little | 25-45% | 60-80% | 30-60 degrees | 31-90 degrees |

The invention may also be embodied in many ways other than those specifically described herein, without departing from the scope thereof.

## Claims

1. A surgical glove hand mould comprising a set of digits, consisting of thumb and
index, middle, ring and little fingers, which are disposed in positions consistent with that of a human hand when holding a surgical implement.

2. A surgical glove hand mould according to claim 1, wherein each of the digits includes at least one major bend.

3. A surgical glove according to claim 1 or claim 2, wherein a first bend of the fingers is located at a proximal-middle joint.

4. A surgical glove hand mould according to claim 3, wherein the first bend is defined as per the following table:
| | Location of Bend 1 | Angle of Bend (a) |
|---|---|---|
| Index | 20-40% | 35-65 degrees |
| Middle | 25-45% | 35-65 degrees |
| Ring | 25-45% | 35-65 degrees |
| Litde | 25-45% | 35-65 degrees |
wherein the location of Bend 1 is defined as the point of the first bend measured from the base of the finger crouch as a percentage of the total length of the straightened finger from finger crouch to fingertip,
wherein the angle of bend (a) of the first bend is defined as the acute angle formed by the intersection of a first line formed along the proximal bone and a second line formed along the middle bone.

5. A surgical glove hand mould according to any one of claims 2 to 4, wherein the first bend of the thumb is located at a metacarpal-proximal joint,
wherein the first bend of the thumb is defined as per the following table:
| | Location of Bend 1 | Angle of Bend (a) |
|---|---|---|
| Thumb | 0-40% | 0-30 degrees |
wherein the location of Bend 1 of the first bend is defined as the point of the first bend measured from the base of the finger crouch as a percentage of the total length of the straightened finger from finger crouch to thumb tip,
wherein the angle of bend (a) of the first bend is defined as the acute angle formed by the intersection of a first line formed along the metacarpal bone and a second line formed along the proximal bone.

6. A surgical glove hand mould according to claim 1, wherein each of the digits includes two bends.

7. A surgical glove according to claim 6, wherein a first bend of the fingers is located at a proximal-middle joint and a second bend of the fingers is located at a middle-distal joint.

8. A surgical glove hand mould according to claim 6 or claim 7, wherein the two bends are defined as per the following table:
| | location of Bend 1 | Location of Bend 2 | Angle of Bend (b) | Combined Aggregate Angle of Bend (c) from the Metacarpal Bone Line |
|---|---|---|---|---|
| Index | 25-45% | 60-80% | 30-60 degrees | 31-90 degrees |
| Middle | 25-45% | 60-80% | 30-60 degrees | 31-90 degrees |
| Ring | 25-45% | 60-80% | 30-60 degrees | 31-90 degrees |
| Little | 25-45% | 60-80% | 30-60 degrees | 31-90 degrees |
wherein the location of Bend 2 is defined as the point of the second bend measured from the base of the finger crouch as a percentage of the total length of the straightened finger from finger crouch to fingertip,
wherein the angle of bend (b) of the second bend is defined as the acute angle formed by the intersection of a first line formed along the middle bone and a second line formed along the distal bone.

9. A surgical glove hand mould according to any one of claims 6 to 8, wherein the first bend of the thumb is located at a metacarpal-proximal joint and a second bend of the thumb is located at a proximal-distal joint, wherein the two bends of the thumb are defined as per the following table:
| | Location of Bend 1 | Location of Bend 2 | Angle of Bend (b) | Combined Aggregate Angle of Bend (c) from the Metacarpal Bone Line |
|---|---|---|---|---|
| Thumb | 0-45% | 0-80% | 0-30 degrees | 0-45 degrees |
wherein the location of Bend 2 is defined as the point of the second bend measured from the base of the finger crouch as a percentage of the total length of the straightened finger from finger crouch to thumb tip,
wherein the angle of bend (b) of the second bend is defined as the acute angle formed by the intersection of a first fine formed along the proximal bone and a second line formed along the distal bone.

10. A surgical glove hand mould according to any one of the preceding claims, wherein the thumb and index finger are dosed to define a gap between them of approximately 1 cm.

11. A surgical glove formed using the mould according to any one of the preceding claims.
